# EUROPEAN PATENT SPECIFICATION

(11) **EP 1 887 973 B1**
(45) Date of publication and mention of the grant of the patent: **19.09.2012**
(21) Application number: 06740140.6
(22) Date of filing: 29.03.2006
(51) Int. Cl.: A61F 2/82

(54) **SELECTIVE TREATMENT OF STENT SIDE BRANCH PETALS**
GEZIELTE BEHANDLUNG VON SEITENZWEIGBLÄTTERN VON STENTS
TRAITEMENT SÉLECTIF DE PÉTALES DE BRANCHE LATÉRALE DE STENT

(30) Priority: 26.05.2005 US 138202
(43) Date of publication of application: 20.02.2008
(73) Proprietor: Boston Scientific Limited, Seaston House Christ Church (BB)
(72) Inventor: MEYER, Michael, P., Richfield, MN 55423 (US); GREGORICH, Daniel, St. Louis Park, Minnesota 55416 (US); GROTHEIM, Kevin P., St. Paul, Minnesota 55114 (US)
(74) Representative: Schildberg, Peter
(86) International application number: PCT/US2006/011816
(87) International publication number: WO 2006/127126

(56) References cited:
- US-A1- 2003 028 233
- US-A1- 2005 060 027
- US-A1- 2005 102 023
- US-B1- 6 835 203

## Description

### BACKGROUND OF THE INVENTION

### Field of the Invention

This invention relates to implantable medical steut.

### Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, fallopian tubes, coronary vessels, secondary vessels, etc. Stents may be used to reinforce body vessels and to prevent restenosis following angioplasty in the vascular system. They may be self-expanding, expanded by an internal radial force, such as when mounted on a balloon, or a combination of self-expanding and balloon expandable (hybrid expandable).

Stents may be created by methods including cutting or etching a design from a tubular stock, from a flat sheet which is cut or etched and which is subsequently rolled or from one or more interwoven wires or braids.

Within the vasculature, it is not uncommon for stenoses to form at a vessel bifurcation. A bifurcation is an area of the vasculature or other portion of the body where a first (or parent) vessel is bifurcated into two or more branch vessels. Where a stenotic lesion or lesions form at such a bifurcation, the lesion(s) can affect only one of the vessels (i.e., either of the branch vessels or the parent vessel), two of the vessels or all three vessels. Many prior art stents however are not wholly satisfactory for use where the site of desired application of the stent is juxtaposed or extends across a bifurcation in an artery or vein such, for example, as the bifurcation in the mammalian aortic artery into the common iliac arteries.

Stents made for use in bifurcated regions are generally known. When treating a bifurcated vessel, it may desirable to use a stent having a side branch opening configured to provide fluid communication between the primary vessel and a secondary or branch vessel of the bifurcation. A secondary or branch stent may be received within and/or be positioned adjacent to the side branch opening of the primary stent.

US 2005/0102023 A1 discloses a stent with a protruding branch portion being deployable outwardly from a stent body into a branch vessel. The branch portion includes three interconnected rings wherein an inner ring defines undulation petals surrounding a central branch opening.

In some locations, a branch vessel may be oriented at an acute angle with respect to a primary vessel. When a stent includes portions which extend into both primary vessels and branch vessels, select elements of the stent may be subject to relatively large amounts of bending, stress and strain.

There remains a need for stents designed for use in bifurcated vessels.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

The invention is directed to a stent comprising a plurality of interconnected strut members. A plurality of the interconnected strut members comprise a plurality of petals, including a first petal. The plurality of petals define a side branch cell. The first petal includes a bending region, wherein the bending region is selectively treated to reduce the strength of the bending region when compared to a region of the first petal adjacent to the bending region. Selective treatment of the bending region may include notching, perforating, scoring, otherwise reducing the cross-sectional area of the bending region, heat treatment, etc.

### BRIEF DESCRIPTION OF THE SEVERAL VIEWS OF THE DRAWING(S)

A detailed description of the invention is hereafter described with specific reference being made to the drawings.
Figure 1 shows a stent having side branch petals.
Figure 2 shows a stent having side branch petals in a bifurcated vessel.
Figure 3 shows an embodiment of a flat pattern for a stent having side branch petals and bending regions.
Figure 4 shows a strut member with various embodiments of bending regions.
Figure 5 shows an embodiment of a petal having multiple bending regions.
Figure 6 shows an embodiment of a strut having multiple bending regions in proximity to one another.
Figure 7 shows the strut of Figure 6 in a deployed configuration.
Figure 8 shows an embodiment of a flat pattern for a stent having side branch petals and bending regions.
Figure 9 shows another embodiment of a flat pattern for a stent having side branch petals and bending regions.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated. Use of the term "parallel" is intended to describe an orientation in which two elements may be exactly parallel or substantially parallel to one another.

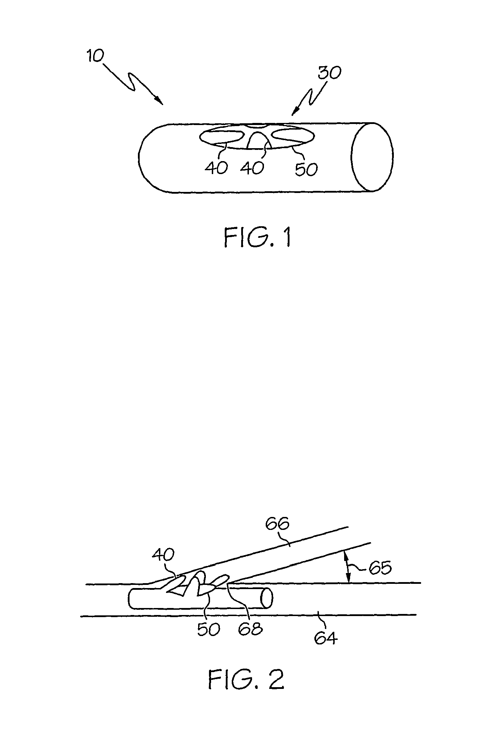

Figures 1 and 2 show an embodiment of a stent 10 having a side branch cell 30 comprising a plurality of petals 40. Select petals 40 may include an inventive bending region 50 as described in detail below. The stent 10 may be positioned within a bifurcated vessel having a main branch vessel 64 and a side branch vessel 66. The stent 10 may be expanded and the petals 40 may deploy outwardly into the side branch vessel 66.

A main branch vessel 64 and a side branch vessel 66 may intersect at an angle 65, and in some instances the angle may be relatively small, for example 45° or less. Petals 40 located to the inside of the bifurcation, for example in proximity to the carina 68, may experience the greatest amount of bending during outward deployment. High amounts of bending may lead to high strains and/or less predictable stent geometry in the fully deployed state. A bending region 50 on select petals 40 is treated to allow the petal 40 to bend predictably at the bending region 50.

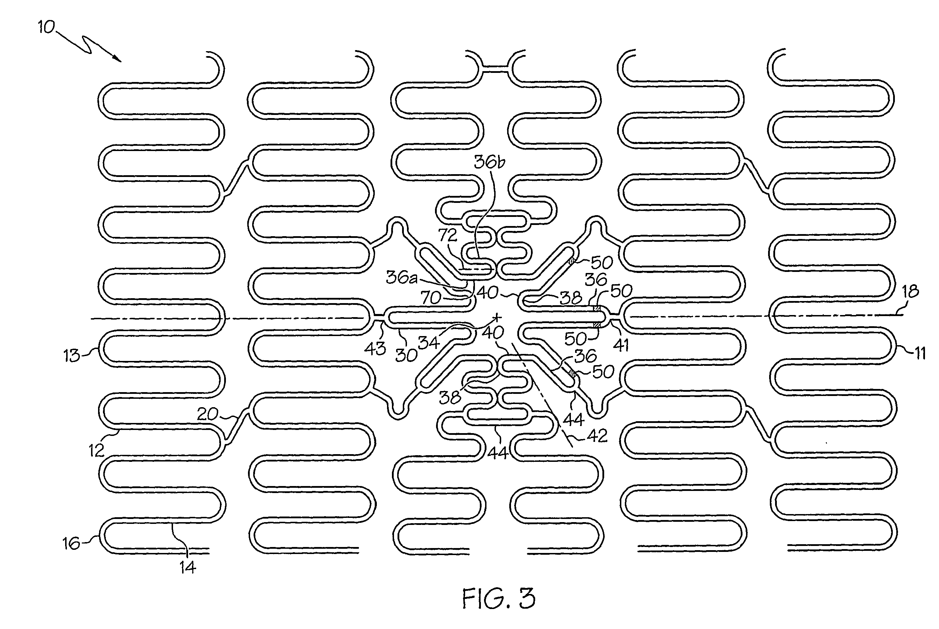

Figure 3 shows a flat pattern for an embodiment of a stent 10 having a side branch cell 30. Some further examples of stent patterns which may be suitable for use in various embodiments of the invention are described in US 5,922,021, US 6,123,721, US 6,334,870, US 6,478,816, US 6,348,065, US 6,325,826.

A stent 10 comprises a proximal end 11 and a distal end 13. The stent 10 may further comprise a plurality of serpentine bands 12 which may have any suitable shape, and comprises plurality of struts 14 connected by turns 16. Adjacent serpentine bands 12 may be connected by connectors 20.

A side branch cell 30 comprises a plurality of side branch petals 40 which may have any suitable shape and may each be oriented in any suitable direction. A side branch cell 30, has any suitable number of petals 40 and in some embodiments may have anywhere from four to fourteen petals 40.

Each petal 40 may have an approximate longitudinal axis 42. In some embodiments, a petal 40 may have a longitudinal axis 42 which is oriented to extend substantially radially outwardly from the center 34 of the side branch cell 30. A longitudinal axis 42 may pass through the centroid of the stent elements which comprise the petal 40.

Each petal 40 may comprise a plurality of struts 36 and at least one turn 38. A strut 36 may be straight along its length, and may be oriented in any suitable direction. A turn 38 may be oriented in any suitable direction. In some embodiments, a turn 38 may be oriented with a peak facing the proximal end 11 of the stent 10, or facing the distal end 13 of the stent 10. Petals 40 which are adjacent to one another about the side branch cell 30 may be connected to one another by a connecting portion 44. In various locations, a connecting portion 44 may comprise a turn 38, a strut 36, or any combination of one or more turns 38 and one or more struts 36.

A petal 40 may include struts 36 that are oriented substantially parallel to the longitudinal axis 18 of the stent 10, and/or may include struts 36 that are oriented substantially parallel to the longitudinal axis 42 of the petal 40. In some embodiments, one or more struts 36 may be oriented at a range from 30° to 60° with respect to the longitudinal axis 18 of the stent 10. In some embodiments, one or more struts 36 may be oriented at approximately 45° with respect to the longitudinal axis 18 of the stent 10. In some embodiments, a petal may include a plurality of struts 36 that are oriented parallel to the longitudinal axis 18 of the stent 10. This may be true even though the longitudinal axis 42 of the petal 40 may be oriented at an angle with respect to the longitudinal axis 18 of the stent 10. In some embodiments, a majority of the struts 36 or all of the struts 36 in a petal 40 may be oriented parallel to the longitudinal axis 18 of the stent 10.

Each petal 40 may further comprise one or more appendages 70. An appendage 70 may comprise a first strut 36a and a second strut 36b connected by a turn 38. An appendage 70 may have an approximate longitudinal axis 72, and the approximate longitudinal axis 72 may be parallel to the longitudinal axis 18 of the stent 10.

Any petal 40 included in a side branch cell 30 includes bending region 50 which is treated to encourage bending in a predetermined fashion. Petals 40 located near a proximal extremity 41 or a distal extremity 43 of the side branch cell 30 may be more likely to include a bending region 50, as these petals 40 are more likely to require high amounts of bending when deployed near a carina 68 (see Figure 2).

A bending region 50 may be located anywhere on a petal 40 and is selectively treated in any suitable fashion to encourage bending of the petal 40 at the bending region 50. A bending region 50 has reduced strength in bending when compared to other regions of the petal 40. In some embodiments, a bending region 50 may have a reduced cross-sectional area, may have indentations and/or perforations, may be chemically treated or heat treated, etc.

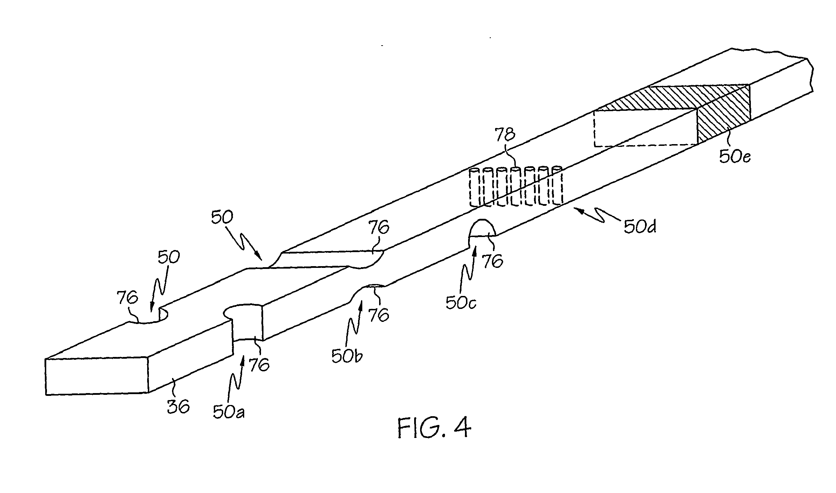

Figure 4 shows a strut 36 having various embodiments of a bending region 50. While a strut 36 is shown, any portion of a side branch cell 30 may include a bending region 50.

In some embodiments, a bending region 50 may have a reduced cross-sectional area when compared to other areas of the strut 36. A bending region 50 may be selectively thinned and may comprise a notch 76 or a plurality of notches 76 which may reduce the cross-sectional area of the strut 36. Notches 76 may be any size or shape, and may have any orientation on the strut 36. In one embodiment, a bending region 50a may include notches 76 in the sides of a strut 36. In one embodiment, a bending region 50b may include notches 76 in the inner and outer surfaces of the strut 36. In another embodiment, a bending region 50c may include a notch 76 in the inner surface of the strut 36. In some embodiments, a notch 76 may extend more than halfway through the thickness of the strut 36. In some embodiments, a bending region 50 may comprise an indentation in a surface of the strut 36.

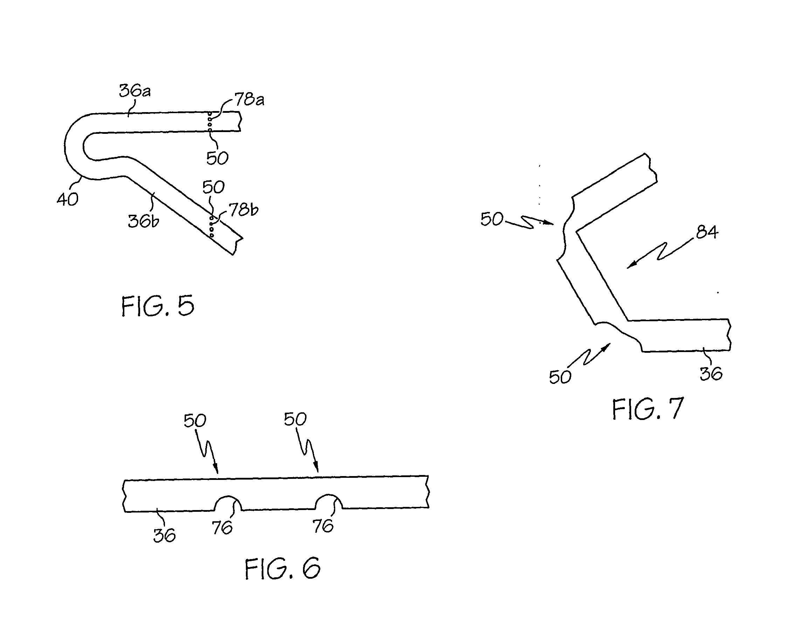

In some embodiments, a bending region 50 may comprise an aperture or perforation 78 which may extend into a strut 36. A perforation 78 may extend through a portion of strut 36 thickness or may extend through the full strut 36 thickness. A perforation 78 may comprise any suitable shape and be oriented in any suitable direction. Thus, a perforation 78 may pass across the thickness of the strut 36, across the width of the strut 36, or may have any other suitable orientation. In some embodiments, a perforation 78 may be laser cut. In some embodiments, a bending region 50d may comprise a plurality of perforations 78 or apertures that are aligned to form a bending plane across which bending of the strut 36 is encouraged. In some embodiments, a plurality of perforations 78 may be aligned across the width of a strut 36. In some embodiments, it may be desirable for a plurality of perforations 78 to be aligned in a direction which crosses a strut 36 width diagonally. For example, Figure 5 shows a petal 40 comprising a first strut 36a and a second strut 36b, wherein the first strut 36a is oriented at an angle with respect to the second strut 36b. A first plurality of perforations 78a may cross the first strut 36a across the width. A second plurality of perforations 78b may cross the second strut 36b diagonally across the width. Cooperation between the first plurality of perforations 78a and the second plurality of perforations 78b may encourage the petal 40 to deploy in a predetermined configuration. In some embodiments, a first plurality of perforations 78a and a second plurality of perforations 78b may share a common axis.

Referring again to Figure 4, another embodiment of a bending region 50e is shown, which may be heat-treated to change properties of the material, reduce strength and/or to allow higher amounts of strain under a given amount of loading. Heat treating, such as annealing, may soften the material in a bending region 50e. The bending region 50e may be heated to a temperature that causes grain growth to occur. A bending region 50e may have a lower yield strength and increased ductility when compared to adjacent areas of the stent, or when compared to the area of the heat treated region before heat treatment. Thus, a bending region 50e may deform at a lower applied stress, may have a higher plasticity and may be capable of accumulating higher amounts of strain than other portions of the stent.

For example, a stent 10 may generally comprise a material having an ASTM E112 grain size #G of 7.0. Such a material may generally have a yield strength of 45 ksi, and a ductility of 50% elongation to fracture. Heat treating the material to form a bending region 50e may change the ASTM E112 grain size #G to a lower grain size, for example a range from 2.0 to 5.0. The bending region 50e material after heat treatment, in some embodiments, may have a yield strength of 20-30 Ksi, and a ductility of 55-70% elongation to fracture.

Heat may be applied to localized regions of the stent using any suitable method, including laser heating, quartz light heating, radio frequency induction heating, etc. In some embodiments, areas outside of the bending region 50e may be masked to protect the outside areas from being heated. Masking may include any suitable type of masking given the method of heating, such as insulative masking, reflective masking, etc.

Further examples of heat treating which may be suitable for use with the invention are disclosed in US Patent Application No. 10/961,289.

In some embodiments, a side branch cell 30 includs a plurality of petals 40, and any of the petals 40 includes a plurality of bending regions 50. Figure 5 shows a petal 40 having a plurality of bending regions 50.

Each bending region 50 included in a side branch cell 30 may comprise any embodiment of a bending region 50. In some embodiments, individual petals 40 may each include a different type of bending region 50. In some embodiments, different types of bending regions 50 may be included in a single petal 40.

Figure 6 shows an embodiment of a strut 36 having two bending regions 50 in proximity to one another. For example, in some embodiments, two bending regions 50 may be in proximity to one another when the distance between the two bending regions 50 is less than two times the width of a strut 36. Each bending region 50 may comprise a notch 76. Upon deployment of the petal which includes the strut 36, the two bending regions may cooperate to allow a two-stage bend or a gradual bend 84 as shown in Figure 7. When the bend 84 in a strut comprises more than one bending region 50, the multiple bending regions may share the strain deformation associated with the bend 84, thereby distributing the strain experienced across the multiple bending regions 50, and reducing the possibly of stress fractures.

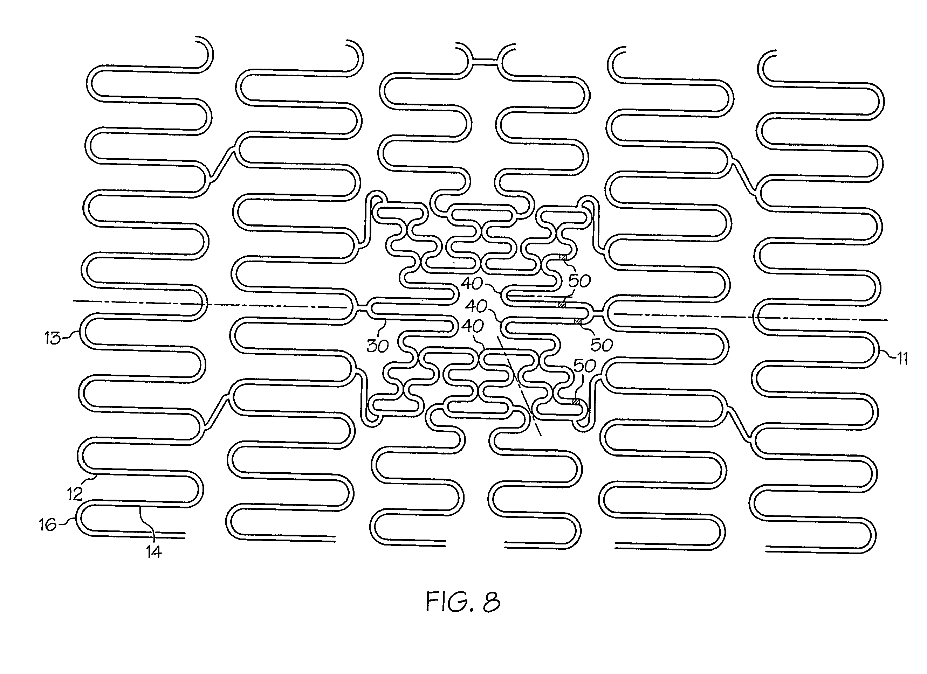

Figure 8 shows another embodiment of a stent 10 having a side branch cell 30 comprising a plurality of petals 40. Each petal 40 includes at least one bending region 50 as herein described.

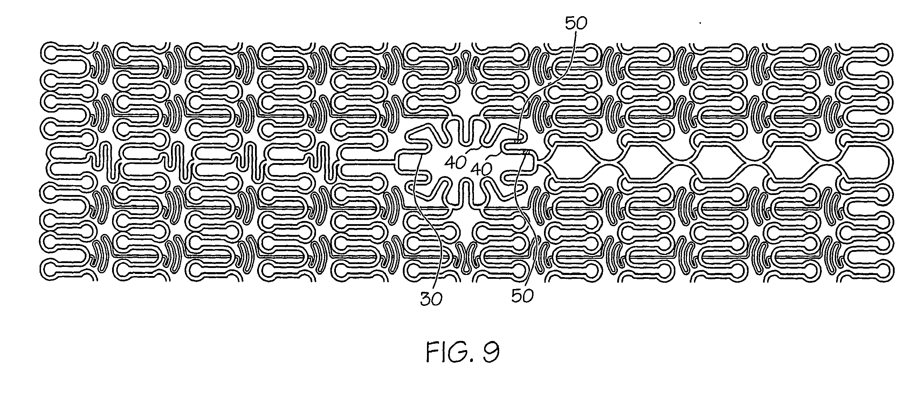

Figure 9 shows another embodiment of a stent 10 having a side branch cell 30 comprising a plurality of petals 40. Each petal 40 includes at least one bending region 50 as herein described.

It is common for a stent to be delivered to a deployment site in a reduced or crimped configuration. The stent may be expanded to a larger diameter, and the petals 40 of a side branch cell 30 may be deployed outwardly. The shape of a side branch cell 30, and the shape of each petal 40 within the side branch cell 30, may change as the stent is expanded from the crimped configuration to the expanded configuration. The bending regions 50 included in a petal 40 may be positioned to affect a predetermined deployment of the petals 40 from any configuration. Therefore, some embodiments may be configured for optimum deployment when the stent is in an expanded state.

In some embodiments, a stent 10, side branch cell 30, or individual petals 40 may include a graft material. For example, a PTFE sheet may be grafted over portions of the stent 10. When a petal 40 includes a graft material which may engage and hold portions of the petal 40, a bending region is be designed to allow a complete detachment of the material on either side of the bending region 50, leaving the petal 40 elements coupled by the graft material. This may be desirable in bifurcations of extreme intersection angles, as the detachment across the bending region 50 may prevent stressing portions of the bifurcated vessels, particularly the carina.

The inventive stents may be made from any suitable biocompatible materials including one or more polymers, one or more metals or combinations of polymer(s) and metal(s). Examples of suitable materials include biodegradable materials that are also biocompatible. By biodegradable is meant that a material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, co-polymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and alloys of any of the above-mentioned metals. Examples of suitable alloys include platinum-iridium alloys, cobalt-chromium alloys including Elgiloy and Phynox, MP35N alloy and nickel-titanium alloys, for example, Nitinol.

The inventive stents may be made of shape memory materials such as superelastic Nitinol or spring steel, or may be made of materials which are plastically deformable. In the case of shape memory materials, the stent may be provided with a memorized shape and then deformed to a reduced diameter shape. The stent may restore itself to its memorized shape upon being heated to a transition temperature and having any restraints removed therefrom.

The inventive stents may be created by methods including cutting or etching a design from a tubular stock, from a flat sheet which is cut or etched and which is subsequently rolled or from one or more interwoven wires or braids. Any other suitable technique which is known in the art or which is subsequently developed may also be used to manufacture the inventive stents disclosed herein.

In some embodiments the stent, the delivery system or other portion of the assembly may include one or more areas, bands, coatings, members, etc. that is (are) detectable by imaging modalities such as X-Ray, MRI, ultrasound, etc. In some embodiments at least a portion of the stent and/or adjacent assembly is at least partially radiopaque.

In some embodiments, at least a portion of the stent is configured to include one or more mechanisms for the delivery of a therapeutic agent. Often the agent will be in the form of a coating or other layer (or layers) of material placed on a surface region of the stent, which is adapted to be released at the site of the stent's implantation or areas adjacent thereto.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

## Claims

1. A stent (10) comprising a plurality of interconnected strut members, a plurality of said interconnected strut members comprising a plurality of petals (40) including a first petal (40), the plurality of petals (40) defining a side branch cell (30), **characterized in that** the first petal (40) includes a bending region (50), the bending region (50) being selectively treated to reduce the strength of the bending region (50) when compared to a region of the first petal (40) adjacent to the bending region (50).

2. The stent of claim 1, wherein said bending region (50) includes less stent material than other portions of the first petal (40).

3. The stent of claim 1, wherein said bending region (50) includes an aperture.

4. The stent of claim 1, wherein said bending region includes a plurality of perforations (78).

5. The stent of claim 4, wherein said perforations (78) are aligned in a direction across said bending region (50).

6. The stent of claim 1, wherein said bending region (50) includes an indentation in a surface of said bending region (50).

7. The stent of claim 6, wherein said indentation is made in an interior surface of the stent.

8. The stent of claim 1, wherein said bending region (50) is heat treated.

9. The stent of claim 1, wherein the first petal (40) is located near an axial extremity of the side branch cell (30).

10. The stent of claim 1, wherein a cross-sectional area of the bending region (50) is less than a cross-sectional area of the region of the first petal (40) adjacent to the bending region (50).

11. The stent of claim 1, wherein the bending region (50) comprises a first bending region (50), the first petal (40) further comprises a second bending region (50), the second bending region (50) selectively treated similar to the first bending region (50).

12. The stent of claim 1, wherein each petal (40) comprises at least one bending region (50), each bending region being selectively treated to reduce the strength of the bending region (50) when compared to a region of the stent (10) adjacent to the bending region (50).

## Patentansprüche

1. Stent (10), umfassend mehrere miteinander verbundene Strebenelemente, wobei mehrere der miteinander verbundenen Strebenelemente mehrere Blätter (40) umfassen, die ein erstes Blatt (40) umfassen, wobei die mehreren Blätter (40) eine Seitenzweigzelle (30) bestimmen, **dadurch gekennzeichnet, dass** das erste Blatt (40) einen Biegebereich (50) umfasst, wobei der Biegebereich (50) gezielt behandelt wird, um die Festigkeit des Biegebereichs (50) im Vergleich zu einem Bereich des ersten Blatts (40), das benachbart zum Biegebereich (50) ist, zu vermindern.

2. Stent nach Anspruch 1, wobei der Biegebereich (50) weniger Stentmaterial umfasst, als andere Abschnitte des ersten Blatts (40).

3. Stent nach Anspruch 1, wobei der Biegebereich (50) eine Öffnung umfasst.

4. Stent nach Anspruch 1, wobei der Biegebereich mehrere Perforationen (78) umfasst.

5. Stent nach Anspruch 4, wobei die Perforationen (78) in einer Richtung über den Biegebereich (50) ausgerichtet sind.

6. Stent nach Anspruch 1, wobei der Biegebereich (50) eine Einkerbung in einer Oberfläche des Biegebereichs (50) umfasst.

7. Stent nach Anspruch 6, wobei die Einkerbung in einer Innenfläche des Stents vorgenommen ist.

8. Stent nach Anspruch 1, wobei der Biegebereich (50) wärmebehandelt ist.

9. Stent nach Anspruch 1, wobei sich das erste Blatt (40) neben einem axialen äußersten Ende der Seitenzweigzelle (30) befindet.

10. Stent nach Anspruch 1, wobei eine Querschnittfläche des Biegebereichs (50) geringer ist, als eine Querschnittfläche des Bereichs des ersten Blatts (40), das benachbart zu den Biegebereich (50) ist.

11. Stent nach Anspruch 1, wobei der Biegebereich (50) einen ersten Biegebereich (50) umfasst, das erste Blatt (40) überdies einen zweiten Biegebereich (50) umfasst, wobei der zweite Biegebereich (50) ähnlich dem ersten Biegebereich (50) gezielt behandelt ist.

12. Stent nach Anspruch 1, wobei jedes Blatt (40) mindestens einen Biegebereich (50) umfasst, wobei jeder Biegebereich gezielt behandelt ist, um die Festigkeit des Biegebereichs (50) im Vergleich zu einem Bereich des Stents (10) zu vermindern, der benachbart zu den Biegebereich (50) ist.

## Revendications

1. Stent (10), comprenant une pluralité d'éléments de montants interconnectés, une pluralité desdits éléments de montants interconnectés comprenant une pluralité de pétales (40) incluant un premier pétale (40), la pluralité de pétales (40) définissant une cellule de branche latérale (30), **caractérisé en ce que** le premier pétale (40) inclut une région de flexion (50), la région de flexion (50) étant sélectivement traitée pour réduire la résistance de la région de flexion (50) quand on compare avec une région du premier pétale (40) adjacente à la région de flexion (50).

2. Stent selon la revendication 1, dans lequel ladite région de flexion (50) inclut moins de matériau de stent que d'autres portions du premier pétale (40).

3. Stent selon la revendication 1, dans lequel ladite région de flexion (50) inclut un orifice.

4. Stent selon la revendication 1, dans lequel ladite région de flexion inclut une pluralité de perforations (78).

5. Stent selon la revendication 4, dans lequel lesdites perforations (78) sont alignées dans une direction à travers ladite région de flexion (50).

6. Stent selon la revendication 1, dans lequel ladite région de flexion (50) inclut une indentation dans une surface de ladite région de flexion (50).

7. Stent selon la revendication 6, dans lequel ladite indentation est réalisée dans une surface intérieure du stent.

8. Stent selon la revendication 1, dans lequel ladite région de flexion (50) est traitée à chaud.

9. Stent selon la revendication 1, dans lequel le premier pétale (40) est situé près d'une extrémité axiale de la cellule de branche latérale (30).

10. Stent selon la revendication 1, dans lequel une surface de section transversale de la région de flexion (50) est plus petite qu'une surface de section transversale de la région du premier pétale (40) adjacente à la région de flexion (50).

11. Stent selon la revendication 1, dans lequel la région de flexion (50) comprend une première région de flexion (50), le premier pétale (40) comprenant également une deuxième région de flexion (50), la deuxième région de flexion (50) étant traitée sélectivement de façon similaire à la première région de flexion (50).

12. Stent selon la revendication 1, dans lequel chaque pétale (40) comprend au moins une région de flexion (50), chaque région de flexion étant traitée sélectivement pour réduire la résistance de la région de flexion (50) quand on compare avec une région du stent (10) adjacente à la région de flexion (50).
